# EUROPEAN PATENT APPLICATION

(11) **EP 3 885 336 A1**
(43) Date of publication of application: **29.09.2021**
(21) Application number: 21162989.4
(22) Date of filing: 28.03.2018
(51) Int. Cl.: C07C 31/10, C07C 29/60, C07C 41/09

(54) **INTEGRATED PROCESS FOR THE PRODUCTION OF FUEL COMPONENTS FROM GLYCERIN**

(30) Priority: 29.03.2017 IT 201700034590
(62) Divisional of application: 18714750.9
(71) Applicant: ENI S.p.A., 00144 Roma (IT)
(72) Inventor: CALEMMA, Vincenzo, 20097 SAN DONATO MILANESE (MI) (IT); ASSANELLI, Giulio, 27100 PAVIA (IT)
(74) Representative: Cernuzzi, Daniele

(57) **Abstract**

The present invention relates to a versatile process for the simultaneous production of biocomponents for fuels such as gasoline and diesel, essentially starting from glycerol of biological origin only, comprising a multiplicity of interconnected steps related to hydrogenation, condensation and etherification.

## Description

The present invention relates to a process for the production of fuel components from glycerin.

In particular, the present invention relates to a balanced process for the simultaneous production of bio-components for gasolines and diesel fuels starting from precursors of biological origin, such as starting from glycerol of biological origin.

With the process according to the present invention it is possible, for example, to produce etherified cyclic acetals having a 1,3-dioxolane structure, to be used as components of diesel fuel, as well as ethers and alcohols to be used as bio components for gasolines substantially starting from glycerol of biological origin.

As is known, the accumulation in the atmosphere of carbon dioxide (CO₂), carbon monoxide (CO), nitrogen oxides (NOₓ), sulfur oxides (SOₓ), unburned hydrocarbons (HC), volatile organic compounds and particulate matter (PM), present in the emissions produced by the combustion of fossil fuels, is the cause of the persistence and worsening of environmental problems, such as acid rain and global warming due to the greenhouse effect.

Over recent years, greater awareness of environmental problems has led to a focus on so-called biofuels, i.e. fuels from renewable sources and of biological origin deriving, for example, from the treatment of algae, plant biomass, oils and fats of plant or animal origin, etc.

On the other hand, new national and supranational regulations and directives impose, within short time-scales, the increasingly extensive use of the aforementioned biofuels, in particular in the sector of fuels for transport.

In particular, European Directive 2009/28/EC, known as the "Renewable Energy Directive" (RED), includes a 20% objective for the total amount of energy from renewable sources and a 10% objective for the proportion of fuels for transport from renewable sources by 2020, supporting and promoting the production and use of biofuels. Likewise, European Directive 2009/30/EC, known as the "Fuel Quality Directive" (FQD), requires that fuel manufacturers reach a 6% reduction in greenhouse gas components in fuels for transport, relative to the reference value of 2010. It is clear that this result can only be reached using formulations of fossil fuels with increasing quantities of biofuel additives or other components deriving from renewable sources.

Among biofuels, the most important are biodiesel and HVO (or green diesel).

Biodiesel is a biofuel is composed of mono alkyl esters of long chain fatty acids derived from vegetable oils or animal fats and that meets the international standard ASTM D6751-15c and falls within the specifications of European standard EN 14214:2008. Pure biodiesel (B100) does not contain derivatives of crude oil, however it is common practice to produce and use biodiesel mixed with diesel of fossil origin, e.g. in percentages of 2 to 10% by volume.

Pure biodiesel can be produced starting from raw materials of biological origin containing triglycerides (triesters of glycerol with long alkyl chain fatty acids). These are subjected to transesterification with a short chain alcohol, in the presence of a catalyst. When the alcohol used in the transesterification is methanol, fatty acid methyl esters (FAMEs) are obtained. Some characteristics of FAMEs make biodiesel a suitable fuel to be used as a mixture component for diesel, e.g. the cetane number, the lubricity, the flash point and the oxygen content are higher with respect to the corresponding fossil fuel, with a substantial absence of aromatic hydrocarbons, in particular polycyclic aromatic hydrocarbons (PAHs) and sulfur compounds. On the other hand, due to its chemical composition, biodiesel comprising FAMEs has some drawbacks connected with the lower oxidation stability and degradation processes caused by bacterial proliferation. The oxygen contained in FAMEs can generate peroxides, which can catalyze the uncontrolled formation of insoluble condensed products such as rubbers, bitumens and other insoluble compounds, which are deposited on filters and on other parts of the engine. Furthermore, the addition of FAMEs to the diesel worsens the cold characteristics and increases the emission of NOₓ by combustion.

The main problems connected with the use of FAME-based biodiesel are described in the Concawe report no. 9/09 "Guidelines for handling and blending FAME" dated November 2009.

Another drawback connected with the production process of FAME-based biodiesel is represented by the fact that it is obtained as a sub-product of glycerol, high quantities of which, in a percentage of about 10% by weight relative to the FAMEs produced, have led to the substantial saturation of the market.

An alternative process for producing biofuels from renewable sources exploits the hydrotreatment of vegetable oils to obtain the so-called HVOs (Hydrotreated Vegetable Oils). HVOs comprise linear paraffin chains or low branch grade chains, free from aromatic hydrocarbons and PAHs, oxygen and sulfur, characterized by a high cetane number. HVOs, which possess the characteristics of a diesel fuel (and in fact constitute so-called Green Diesel), can be obtained by the hydrodeoxygenation of a material of biological origin, e.g. palm oil, soy oil, rape seed oil, corn oil, sunflower oil, comprising triglycerides and possibly free fatty acids, in the presence of hydrogen and a catalyst, which can optionally be followed by the hydroisomerization of the product obtained, still in the presence of hydrogen and a catalyst, for the purpose of increasing the branched hydrocarbon proportion, as described, for example, in WO 2009/039347 A1.

It is known that HVOs represent an improvement with respect to FAME-based biodiesels, in particular in terms of greater oxidation stability and greater cold properties (Faraci, G., Gosling, C., Holmgren, J., Marinangeli, R., Marker, T., Perego, C., in "New developments in renewable fuels offer more choices", Hydrocarbon Processing, September 2007 issue, pag. 67-71). Furthermore, with respect to FAMEs, HVOs do not have the problem of increasing NOₓ emissions by combustion.

On the other hand, HVOs are characterized by a lower density with respect to diesel of fossil origin and, because of the lack of oxygen atoms in their molecular structure, they do not provide any significant benefits for the purpose of reducing particulate matter when used in diesel engines mixed with diesel, in a lower amount than 5% by volume relative to the total volume of said mixture. To contrast this drawback, it is possible to resort to the addition, to diesel fuels comprising HVOs, of oxygenated compounds that promote the complete combustion of hydrocarbons and therefore reduce the emission of particulate (Chen, H., Wang, J., Shuai, S., Chen, W., "Study of oxygenated biomass fuel blends on a diesel engine" (2008), Fuel, vol. 87, pag. 3462-3468).

The aforementioned oxygenated compounds can be obtained starting from glycerol and in particular glycerol of biological origin has been shown to be an effective expedient for exploiting the fraction of the glycerol itself, obtained in biodiesel production processes in excess with respect to market demand (Garcia, E., Laca, M. Perez, E., Garrido, A., Peinado, J., "New class of acetal derived from glycerin as a biodiesel fuel component" (2008), Energy & Fuels, vol. 22, pag. 4274-4280).

WO 2005/093015 A1 describes a process for the production of biofuels though the transformation of triglycerides into at least two biofuel families containing fatty acid monoesters and ethers and/or soluble glycerol acetals. Said ethers and acetals of the prior art had high water affinity and low miscibility with the hydrocarbon phase. In actual fact, such characteristics limit the use of these compounds as fuel components, since they promote the solubilization of non-negligible quantities of water, also responsible for any corrosion phenomena of the metal parts of the engine.

International patent application WO 2013/150457, filed by the Applicant, describes new fuel compositions comprising hydrophobic derivatives of glycerol, represented by ketals or cyclic acetals of the 1,3-dioxolane and 1,3-dioxane type. Because of their hydrophobic behavior, said compounds provide high performance levels as fuel components as they allow the problems of the acetals previously proposed to be overcome, in relation to their high affinity with water and low affinity with the remaining hydrocarbon component of the fuel, contributing at the same time to a reduction in particulate matter emissions and without significantly altering the actual characteristics of the fuel, e.g. the cloud point (CP), the cold filter plugging point (CFPP), the demulsibility characteristics and the lubricity properties.

International patent application WO 2014/125416, filed by the Applicant, describes an integrated process for the production of fuel components that comprises:
- the transformation of glycerol into an alkoxy-propanediol having formula RO-CH₂-CHOH-CH₂OH, where R is a linear or branched C₁-C₈ alkyl,
- the transformation of glycerol into 1,2-propanediol,
- the dehydration of the 1,2-propanediol obtained to propionic aldehyde,
- the reaction by the propionic aldehyde obtained with alkoxy-propanediol having formula RO-CH₂-CHOH-CH₂OH, to provide an acetal having formula (C): 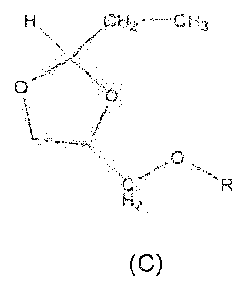 where R is a linear or branched C₁-C₈
- the oxidation by the propionic aldehyde obtained to propionate having formula CH₃-CH₂-COOR', where R' is a linear or branched C₁-C₈ alkyl.

The synthesis of the cyclic acetal takes place through the reaction of an etherified diol with a carbonyl compound, in the case in question propionic aldehyde, which is specifically obtained by dehydration of the 1,2-propanediol.

International patent application WO 2015/155659, filed by the Applicant, describes a process for producing 1,3-dioxolane type cyclic acetals, which can be used, for example, as fuel components having general formula (D): 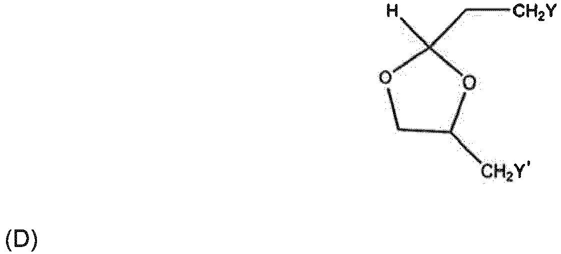 where Y and Y', identical or different, are selected from H and an OR group, R being a linear or branched alkyl containing 1 to 8 carbon atoms. The embodiments of the invention that provide for Y and/or Y' being OR, i.e. those in which the cyclic acetal having formula (D) is etherified are preferred, particularly for use as a diesel fuel component.

The aforementioned process comprises the steps of:
(i) providing a reaction mixture comprising at least one vicinal diol having formula Z-CH₂-CHOH-CH₂OH, where Z is selected from H and an OR' group, R' being a linear or branched alkyl containing 1 to 8 carbon atoms,
(ii) heat treating said reaction mixture to a temperature in the range 100°C-300°C in the presence of at least one acid catalyst.

Although the process described in WO 2015/155659 is characterized by excellent yields, it is particularly simple when a compound is required having formula (D) in which Y is equal to Y'. Otherwise, to obtain compounds having formula (D) in which Y is different from Y', step (i) of the process must provide a reaction mixture having at least two vicinal diols, whose subsequent heat treatment leads to the formation of at least four different compounds having formula (D) in mixture together. In other words, with the process of WO 2015/155659, the yield for obtaining a compound having formula (D) will only be optimal in the cases in which said compound is characterized by a residual Y equal to Y'. On the contrary, the yield of a compound having formula (D) obtained in which the two residues Y and Y' are different will be inversely proportional to the number of compounds having formula (D) with the different combinations of Y and Y' theoretically obtainable through said process.

It is therefore clear that in the case described above, the total yield of the process may be compromised by the fact that undesired compounds having formula (D) are obtained. Furthermore, the isolation from the mixture of products of an individual compound having formula (D) with a different residue Y from the residue Y' could be difficult and imply the adoption of various separation and purification steps.

The aforementioned processes and compositions are essentially aimed at obtaining additive components for diesel and do not consider in the complex the need also to improve the performance levels of fuels having a boiling point in the range of gasolines and fuel oils, typically between 70 and 175°C under normal conditions.

The biofuel from renewable sources commonly used in the gasoline blend is ethanol. It is produced through fermentation processes starting from different materials such as sugar cane, sugar beet; grains such as corn, barley, potatoes, cassava; lignocellulosic plants such as agricultural waste, straw and forest residues. Although ethanol has a series of positive characteristics as a component mixed with the gasoline blend (high octane number, similar density to that of gasoline, complete miscibility with gasoline, presence of oxygen), there is a series of disadvantages that have prevented its wider use (ethanol is miscible with water causing separation phenomena with hydrocarbons, ethanol forms azeotropes with light hydrocarbons causing volatility problems, low calorific power and the high latent heat of vaporization can cause problems for cold starts). Therefore, the need is felt for a biofuel for gasoline blends that solves or mitigates the disadvantages previously mentioned. Propanol has the same positive characteristics as ethanol with reference to its ignition qualities and calorific power, but it has a higher energy density (+12%), lower volatility, and lower latent heat of vaporization (-8%). Therefore, the production of propanol starting from a renewable and cheap source constitutes a solution to the problems mentioned above.

Patent DE102008026583 is also known for the production of 1-propanol or mixtures of 1- and 2-propanol by hydrogenation of glycerin in two steps, to be used as a gasoline additive. However, such publication does not propose a versatile use of the propanol derived from glycerin, such as to adapt to the periodic variations and requirements of the fuel market.

To the best of our knowledge, a process is not known which is able to produce biofuels to be conveniently mixed both with the gasoline blend and the diesel blend, starting from glycerin only.

Therefore, the problem of reallocating the excess of glycerin coming from biofuel production processes remains very salient. The Applicant therefore set out to identify a process that allows on one hand at least one component for mixtures with gasolines and on the other hand at least one component for mixtures with diesels to be obtained simply and with high yields, essentially starting from glycerin as a source of carbon reagent.

In this way, through the process according to the present invention, it is possible to exploit completely, simply and cheaply, the glycerol obtained as a sub-product of transesterification or hydrolysis reactions of the triglycerides contained in lipids of plant or animal origin used in biofuel production processes.

The problem is also solved of the production of propanol as a sub-product in the hydrogenation step of glycerin to 1,2-propanediol.

The Applicant has therefore surprisingly found that the aforementioned drawbacks can be solved overall by identifying a production range of 1,2-propanediol and 1-propanol starting from glycerin, that allows bio-fuels to be obtained both for the diesel or kerosene fuel sector and for the gasoline sector, hence allowing components to be flexibly produced for both sectors with a single process and substantially starting from glycerin only.

The first subject matter of the present invention is a process for producing fuel mixture components for diesel and gasoline starting from glycerin only, preferably coming from processes of biological origin, comprising the following steps:
a) catalytic hydrogenation of glycerin, with a conversion of at least 50% to obtain a mixture of 1-propanol and 1,2-propanediol in a molar ratio to each other from 50/50 to 90/10;
b) separation of 1-propanol, 1,2-propanediol and glycerin from the reaction mixture of step (a);
c) condensation of the 1,2-propanediol obtained in step (b) in the presence of an acid catalyst to obtain 2-ethyl-4-methyl-1,3-dioxolane (PrDIOX);
d) glycerin etherification by reaction with 1-propanol, preferably obtained in the separation step (b), under conditions to obtain predominantly 3-(n-propoxy)-1,2-propanediol;
e) reaction of the 3-(n-propoxy)-1,2-propanediol obtained in step (d) and the 2-ethyl-4-methyl-1,3-dioxolane obtained in step (c) in the presence of an acid catalyst to obtain 2-ethyl-4-(n-propoxymethyl)-1,3-dioxolane;
f) maintaining the remaining part of 1-propanol obtained in step (b) for subsequent uses.

The present invention secondly relates to a method for improving the performance levels of diesel and gasoline fuels through mixing with oxygenated components originating from the transformation of glycerin, preferably coming from processes of biological origin, comprising:
a') catalytic hydrogenation of glycerin, with a conversion of at least 50% to obtain a mixture of 1-propanol and 1,2-propanediol in a molar ratio to each other from 50/50 to 90/10;
b') separation of 1-propanol, 1,2-propanediol and glycerin from the reaction mixture of step (a);
c') condensation of the 1,2-propanediol obtained in step (b) in the presence of an acid catalyst to obtain 2-ethyl-4-methyl-1,3-dioxolane (PrDIOX);
d') glycerin etherification by reaction with the 1-propanol, preferably obtained in the separation step (b), under conditions to obtain predominantly 3-(n-propoxy)-1,2-propanediol;
e') reaction of the 3-(n-propoxy)-1,2-propanediol obtained in step (d) and the 2-ethyl-4-methyl-1,3-dioxolane obtained in step (c) in the presence of an acid catalyst to obtain 2-ethyl-4-(n-propoxymethyl)-1,3-dioxolane;
f') use of the remaining part of the propanol obtained in step (b) as a component, preferably bio-component, for a gasoline fuel mixture;
g') use of the 2-ethyl-4- (n-propoxymethyl)-1,3-dioxolane obtained in step (e) as a component, preferably bio-component, for a diesel fuel mixture.

In accordance with the present invention, the aforementioned steps (a'), (b'), (c'), (d') and (e') of the method for improving the performance levels of diesel and gasoline fuels, can be performed under the same general and particular conditions and the same intermediate products as of the corresponding steps (a), (b), (c), (d) and (e) of the present process for producing mixture components for diesel and gasoline, can be obtained.

In accordance with the aforementioned method for improving the performance levels of fuels, in said steps (f) and (g'), said 1-propanol and 2-ethyl-4-(n-propoxymethyl)-1,3-dioxolane are mixed with gasoline and diesel, respectively, in an amount independently comprised between 0.5% by volume and 15% by volume, preferably comprised between 1% by volume and 10% by volume, relative to the volume of each mixture thus obtained.

For the purpose of the present invention, any hydrocarbon mixture usable as a fuel can be used. In particular, the hydrocarbon mixture can be selected from diesel, gasoline, biodiesel, green diesel, and mixtures thereof.

Other subject matters and characteristics of the present invention shall become clear in the following description and claims.

For the purposes of the present description and following claims, the definitions of the numeric ranges always include the extremes unless specified otherwise. The pressures are always expressed as absolute pressures.

In the description of the embodiments of the present invention, the use of the terms "comprising" and "containing" indicates that the options described, for example regarding the steps of a method or of a process or the components of a product or of a device, are not necessarily all-inclusive. It is however important to note that the present application also relates to the embodiments in which the term "comprising" in relation to the options described, e.g. regarding the steps of a method or of a process or the components of a product or of a device, must be interpreted as "which essentially consists of" or "which consists of", even if this is not explicitly stated.

For the purpose of the present description and following claims, the percentages are always by weight, except in cases in which it is specified otherwise.

For the purposes of the present description and the following claims, every time reference is made to a compound, whether it is a reagent or a product, of which different stereoisomers may exist, for example enantiomers (R) - (S) in the event in which there is at least one asymmetrical sp³ carbon, said reference to said compound comprises all its possible stereoisomers.

For the purposes of the present description and the following claims, "molecule with a 1,3-dioxolane structure" means any cyclic molecule comprising the 1,3-dioxolane nucleus.

For the purposes of the present description and the following claims, "molecule with a 1,3-dioxane structure" means any cyclic molecule comprising the 1,3-dioxane nucleus. For the purposes of the present description and the following claims, "etherified" compound (e.g. "etherified diol", or "etherified cyclic acetal") means a compound that comprises at least one ether functional group (e.g. OR') in its structural formula, R' being an alkyl having 1 to 6 carbon atoms.

For the purposes of the present description and the following claims, "carbonyl compound" means a compound characterized by a C=O functional group which, when reacted with a diol, can generate an adduct such as an acetal or a ketal. Carbonyl compounds comprise, for example, adhehydes, ketones, urea, alkyl carbonates, dialkyl carbonates, etc.

For the purposes of the present invention, the term "diesel" means a mixture mainly comprised of hydrocarbons such as paraffins, aromatic hydrocarbons and naphthenes, typically having 9 to 30 carbon atoms, which can be used as fuel. Generally, the distillation temperature of diesel is comprised between 180°C and 450°C. Said diesel can be selected either from diesels that fall within the specifications of diesel for transport according to standard EN 590:2009 or those for diesels that do not fall within said specifications. Said diesel may have a density, at 15°C, determined according to standard EN ISO 12185:1996/C1:2001, comprised between 780 kg/m³ and 845 kg/m³, preferably comprised between 800 kg/m³ and 840 kg/m³. Said diesel may have a flash point, according to standard EN ISO 2719:2002, greater than or equal to 55°C, preferably greater than or equal to 65°C. Said diesel may have a cetane number, determined according to standard EN ISO 5165:1998, or standard ASTM D6890:2008, greater than or equal to 47, preferably greater than or equal to 51.

Diesels that can be used successfully for the purposes of the present invention may be all the known ones, possibly deriving from the mixture of diesel blends of different origins and compositions. Preferably the sulfur content of these diesel blends is comprised between 200 and 1 mg/kg, and even more preferably between 10 and 1 mg/kg. Typical diesels may be middle distillates, preferably having a boiling point comprised between 180 and 380 °C, such as diesels from primary distillation, diesels from vacuum distillation, diesels from thermal or catalytic cracking, such as desulfurized diesel from fluid catalytic cracking, light cycle oil (LCO), diesels from a Fischer-Tropsch process or of synthetic origin. The term "diesel" also comprises so-called green diesel and biodiesel blends and mixtures thereof with traditional refinery diesels.

For the purposes of the present description, the term "gasoline" means a mixture prevalently comprising hydrocarbons such as, by way of example, paraffins, aromatic hydrocarbons and naphthenes, typically having from 5 to 12 carbon atoms, which can be used as fuel, characterized by a T95 (ASTM D86) not greater than 250 °C, preferably not greater than 240 °C, where T95 means the temperature at which 95 % by volume of the gasoline is distilled. Said gasoline may have a density comprised between 855 and 910 kg/m³. Usable gasolines are those deriving from catalytic processes, preferably deriving from Fluid Catalytic Cracking (FCC) processes, from reforming processes, and mixtures thereof. In particular, HCN gasolines, i.e. heavy gasolines (initial boiling point 150 °C) from FCC, can be used, as such or desulfurized, and heavy reformed gasolines (initial boiling point 150 °C) from reforming, or mixtures thereof.

Preferably the sulfur content of these gasoline blends is comprised between 50 and 0.1 mg/kg, and even more preferably between 10 and 0.5 mg/kg.

The process in accordance with the present invention therefore allows the complete reconversion of the glycerin derived as a sub-product from animal or plant fat treatment processes in order to obtain biofuels. This does not exclude, for the purposes of the present invention, the glycerin supplied having other origins or sources, such as a synthetic origin.

The glycerin used in the process in accordance with the present invention is supplied in part in the hydrogenation step (a), in part in the etherification step (d) with 1-propanol. A person skilled in the art decides on the relative quantity of glycerin supplied at the two steps based on the desired weight ratio between the two final products of the process, obtained as such or used in mixture with gasoline and diesel, respectively. The glycerin supplied preferably has a commercial purity grade of at least 98%. Otherwise, it can be purified in advance from the excess salts and water that may be present in the event that it is derived from the transesterification of triglycerides. Before the crude glycerin deriving from said processes (80-85%) can be used in the process according to the present invention, it is subjected to a purification pre-treatment to obtain glycerin with the desired degree of purity. Said purification can be performed, for example, through a process comprising two steps:
- in the first step, the salts contained in the crude glycerin are removed, coming from the production of FAME, through treatment on acid exchange resins such as Amberlyst 15, Amberlyst 36, performing the removal preferably at temperatures comprised between 0 and 60°C and even more preferably between 15 and 30°C, operating at atmospheric pressure;
- in the second step the impurities present in the crude glycerin are removed, mainly comprising water with small amounts of methanol, through fractional distillation, until a glycerin content of at least 95-96% is obtained.

Further details related to the purification of glycerin are described, for example, in "PERP Report Glycerin conversion to propylene glycol 06/07S4, March 2008". The glycerin resulting from the steps described above may be used in the process according to the present invention without any further purification.

In accordance with step (a) of the process according to the present invention, the glycerin is hydrogenated with gaseous hydrogen in the presence of a suitable catalyst to obtain a mixture of 1-propanol and 1,2-propanediol in proportions selected so as to obtain at the end of the present process the desired proportion of 1-propanol and 2-ethyl-4-(n-propoxymethyl)-1,3-dioxolane. For example, if the two components are to be obtained in a molar proportion of 1/1, step (a) must be performed so as to produce 1-propanol and 1,2-propanediol in molar proportions of 2/1.

Step (a) can also be performed in a single reactive step, or in two sub-steps in sequence, first substantially producing 1,2-propanediol and subsequently hydrogenating part of this to 1-propanediol. In the case of two sub-steps, they may be performed in two separate reactors or in different zones of the same reactor, in which there are specific conditions and catalysts. Methods, catalysts and process conditions for obtaining in one or two steps high conversions and selectivity as well as the desired ratio between the two products 1-propanol and 1,2-propanediol are known in the state of the art and reported in numerous publications, e.g. in J. Mol. Cat. A Chem., Vol. 365 (2012), pag. 24; Catal. Sci. Technol. 2011, 1, 179-190; Appl. Catal. B: Envir. 2016, 193, 75.

The hydrogenation step (a) of glycerin can be performed, for example, by reacting glycerin with hydrogen in the presence of a reduction catalyst. Reduction catalysts that can be used may be all the known ones. For example, copper chromite, mixed chrome-zinc-copper oxides, noble metals on coal, noble metals on iron oxide, in particular palladium on coal, platinum on coal and palladium on iron oxide.

Step (a) can be performed in glycerin as such, or in the presence of a solvent. Possible solvents that can be used are for example the same reaction products 1-propanol and/or 1,2-propanediol or a mixture thereof, preferably in the same proportions desired for the product of the hydrogenation reaction. Another solvent or co-solvent may be water, which however constitutes a reaction product and is always present in the mixture exiting from step (a). The hydrogenation reaction may be performed at a temperature comprised between 100°C and 250°C, under hydrogen pressure comprised between 0.1 and 10 MPa absolute. The conversion of the glycerin (calculated as a % of reacted glycerin with respect to the glycerin supplied) is at least 50%, preferably from 55% to 99%, more preferably from 60 to 90%.

In the aforementioned conditions, mixtures of 1-propanol and 1,2-propanediol are obtained, containing lower quantities, at the most up to 5% by weight with respect to the weight of 1-propanol and 1,2-propanediol produced, of 2-propanol and 1,3-propanediol. The mixture of reaction products may also comprise acetol in quantities of not more than 15% by weight of the main products, as well as any unreacted glycerin. Other reaction sub-products, such as ethylene glycol, ethanol, propane, may be present in a total amount of less than 5%, preferably less than or equal to 2% by weight with respect to the total weight of propanol and propanediol. Preferably, 1-propanol and 1,2-propanediol are obtained in molar ratios to each other comprised between 60/40 and 80/20, more preferably from 65/35 to 75/25.

The process according to the present invention may be performed using conventional pressure equipment known to a person skilled in the art.

The separation step (b) of the mixture of products obtained in step (a) can normally be performed through fractional distillation, continuously or discontinuously, preferably continuously, using, for example, an appropriately sized fractionating column. The separation step (b) can also be performed through two or more distillation columns, placed in series with each other. The boiling points and phase diagrams of the different compounds and mixtures thereof are well known and adapted to allow separation through a single distillation at least of the main components of the mixture, i.e.: water, 1-propanol with possible smaller amounts of 2-propanol, 1,2-propanediol with possible smaller amounts of 1,3-propanediol, glycerin with any acetal formed as an intermediate. The columns used may be made of stainless steel or other suitable materials according to what is known in the state of the art.

According to a particular aspect of the present invention, the separation of the reaction products of step (a) may be performed using a first column that operates at atmospheric pressure that separates as the last product a high boiling point mixture containing 1,2-propanediol, glycerin and possibly acetol, subsequently separated by atmospheric or vacuum distillation of the propanediol. The stream containing water, n-propanol and isopropanol, as well as any sub-products such as ethanol, are sent, according to a well-known technique applied to similar mixtures obtained in other processes, to a distillation system comprising two interconnected columns that operate at atmospheric pressure and at reduced pressure, known as "pressure swing distillation", which allows 1,2-propanol and 1,3-propanol to be separated from their azeotropic mixture with water.

According to a preferred embodiment of the present invention, the unreacted glycerin separated in step (b) is recycled to step (a), together with any acetol formed as a sub-product.

For the uses of 1-propanol as a mixture component of gasolines, the possible presence of 2-propanol in mixture does not represent any drawback and can also be successfully used as a component for gasolines, without any further separation. Also for the part of 1-propanol used to supply the subsequent esterification step (d), the possible presence of 2-propanol does not represent a drawback, even if the isopropyl ether correspondingly obtained is equally usable in step (e) of the process in accordance with the present invention, for obtaining the corresponding 2-ethyl-4-(isopropoxymethyl)-1,3-dioxolane.

Any 1,3-propanediol formed as a sub-product does not need to be separated from 1,2-propanediol and can be supplied together with the latter to the condensation step (c) and possibly separated later by distillation.

In accordance with step (c) of the present invention, the 1,2-propanediol obtained in the separation step (b) is subjected to a condensation reaction in the presence of a suitable acid catalyst. Such reaction is a self-condensation in which two molecules of 1,2-propanediol react together to provide the cyclic acetal 2-ethyl-4-methyl-1,3-dioxolane. Preferably, said condensation step is performed at a temperature comprised between 100°C and 300°C, more preferably between 110°C and 200°C. In a particularly preferred aspect, said step (c) is performed at a temperature comprised between 115°C and 150°C.

Preferably, said condensation reaction is performed maintaining the reaction mixture in the liquid phase. For that purpose, it is preferable to perform the reaction maintaining the mixture comprising 1,2-propanediol at a pressure comprised between 0.5 MPa and 5 MPa, more preferably at a pressure comprised between 1 MPa and 4 MPa.

Acid catalysts suitable for performing the reaction in step (c) may in general be the heterogeneous acid catalysts normally used in etherification or dehydration reactions of alcohols and diols for obtaining unsaturated compounds. For example, said acid catalyst can be selected from acid ion exchange resins, zeolites in acid form, silico-alumina, supported phosphoric acid and mixtures thereof.

The acid ion exchange resins may be directly used in the form of microspheres, as normally available on the market. The acidic zeolites and silico-alumina are preferably extruded together with a binder.

Acid resins that can be used are those containing sulfonic or carboxylic groups as acid groups.

Commercial resins can be used, such as Amberlyst A-36, Amberlyst A-70, Amberlyst BD-20, Amberlite IR-120, Amberlite IRC-86, Amberlite IRC-50, Nafion.

Preferred zeolites are medium or large pore zeolites, even more preferably, zeolite Y, zeolite Beta or zeolite ZSM-5.

The zeolites are used in acid form, i.e. in the form in which the cation sites present in their structure are occupied at least 50% by hydrogen ions, and it is especially preferred that at least 90% of the cation sites are occupied by hydrogen ions.

Said step (c) can be performed discontinuously or continuously and is preferably performed continuously.

The Liquid Hourly Space Velocity (LHSV) of the reaction mixture on the catalyst is preferably comprised between 0.1 and 20 h⁻¹ and more preferably it is comprised between 1 and 12 h⁻¹. In a particularly preferred aspect, the spatial velocity is comprised between 2 and 8 h⁻¹.

Preferably, the condensation reaction of step (c) is performed in at least one fixed-bed catalytic reactor.

In a preferred aspect, said condensation can be performed in at least one fixed-bed reactor continuously recycling unconverted reagents. The recycling ratio may be comprised in the range between 10 and 25, and is preferably comprised between 15 and 20.

The reaction can be performed with other reaction systems, such as a CSTR (Continuous Stirred-Tank Reactor) or an ebullated bed reactor.

In accordance with step (d) of the process according to the present invention 3-propoxy-1,2-propanediol is obtained through an etherification process with glycerol with 1-propanol obtained after the separation step (b), according to conventional methods, e.g. by reacting the glycerol with 1-propanol in the presence of at least one acid catalyst. Preferably, the 1-propanol used in step (d) comes in part or, more preferably, all from the separation step (b) and therefore constitutes a part of the 1-propanol obtained by hydrogenation of the glycerin in step (a). Alternatively, at least a part of the 1-propanol supplied to step (d) is obtained in a different way, preferably biologically, e.g. from the fermentation of biomass or of derivatives of biomass, in particular of lignocellulosic biomass or algal biomass. The use in step (d) of a part of 1-propanol not obtained from the same glycerin can advantageously allow more effective adjustment and that complies with market requirements of the present process, if appropriate, for example, by using a larger proportion of 1-propanol produced by the hydrogenation of glycerin as a mixture component for gasolines, with respect to what is allowed by the stoichiometry of the process, or by the temporary conditions in which it is performed, without having to change them substantially.

Step (d) is conveniently performed by reacting the glycerin in the presence of 1-propanol and of an acid catalyst and possibly in the presence of a solvent. Acid catalysts that can be successfully used are, for example, acid exchange resins, acidic zeolites, silico-alumina, supported phosphoric acid. The acid exchange resins can be directly used in the form of microspheres, as normally provided by the producer; the acidic zeolites and silico-alumina are preferably extruded using any known binder, for example alumina. Step (d) is preferably performed using the same 1-propanol as the solvent, i.e. in stoichiometric quantities or in reasonable excess relative to the glycerin. In this latter case, a person skilled in the art can assess the excess quantity so as not to promote the formation of etherified glycerin on two of its three oxygenated positions. The alcohol/glycerin molar ratio is preferably comprised between 1 and 5.

Preferably, the reaction is performed at a temperature comprised between 50 and 200°C and at a pressure comprised between 1 and 20 atm. The spatial velocity in the event of a process performed continuously is preferably comprised between 0.1 and 20 hours⁻¹. Etherification in position 1 is preferred, using a fixed-bed reactor, with a choice of low contact times. The supply of the reagents to the reactor can be performed either countercurrent or co-current.

In accordance with step (e) of the process according to the present invention, the reaction is performed, in the presence of an acid catalyst, selected from 3-(n-propoxy)-1,2-propanediol obtained in step (d) and 2-ethyl-4-methyl-1,3-dioxolane obtained in step (c) to obtain 2-ethyl-4-(n-propoxymethyl)-1,3-dioxolane.

In particular, in said step (e) 2-ethyl-4-methyl-1,3-dioxolane is reacted in the presence of an acid catalyst with 3-(n-propoxy)-1,2-propanediol selectively exchanging the diol residue of the dioxolane cycle with vicinal hydroxyls of said 3-(n-propoxy)-1,2-propanediol. In the reaction, 1,2-propanediol is formed as a co-product which is subsequently separated, normally by distillation, and recycled to step (c) to form more 2-ethyl-4-methyl-1,3-dioxolane. Preferably, the reaction is performed in the presence of substantially equimolar quantities of two reagents, but the scope of the present patent application does not exclude the performance of the reaction with different proportions, e.g. in molar ratios between 2-ethyl-4-methyl-1,3-dioxolane and 3-(n-propoxy)-1,2-propanediol comprised between 65/35 and 35/65.

In the event that 3-(n-propoxy)-1,2-propanediol from step (d) is mixed with lower quantities of 2-propoxy-1,3-propanediol, preferably less than 30% by moles, more preferably less than 10% by moles, with respect to the total moles of the two etherified diols, formed as a sub-product, this does not need any separation in accordance with the present invention, but can contribute, by reacting with 2-ethyl-4-methyl-1,3-dioxolane under the same operating conditions as step (e), to the formation of 2-ethyl-5-propoxymethyl-1,3-dioxane, which however constitutes a desirable mixing biocomponent for diesel.

Step (e) of the process according to the present invention can be performed at a temperature comprised between 30°C and 150°C and is preferably performed at a temperature comprised between 40°C and 120°C. In a particularly preferred aspect, said process is performed at a temperature comprised between 40°C and 80°C.

Preferably, said step (e) of the process according to the invention is performed maintaining the reaction mixture in the liquid phase. For that purpose, the process can be performed at a pressure comprised between 0.5 MPa and 5 MPa and is preferably performed at a pressure comprised between 1 MPa and 4 MPa.

The recycling ratio may be comprised in the range between 10 and 25, and is preferably comprised between 15 and 20.

In a preferred aspect, said process can be performed in at least one fixed-bed reactor continuously recycling unconverted reagents.

The acid catalyst used in the process according to the present invention can be selected from acid ion exchange resins, zeolites in acid form, silico-alumina, supported phosphoric acid and mixtures thereof. Suitable acid catalysts are the same ones previously described, in general and in particular, for performing step (c).

It is important to note that the cyclic acetals obtained at the end of steps (c) and (e) of the process according to the present invention, can be in the form of mixtures of different geometric and/or optical isomers. For the purposes of the present invention, either individual isomers or mixtures of said isomers can be used as mixture components for diesel.

It will be clear to a person skilled in the art that the determination in the desired ratio between 1-propanol and 2-ethyl-4-(n-propoxymethyl)-1,3-dioxolane obtained at the end of the present process, automatically determines, considering the losses due to any sub-products, the proportions of the different reagents in the steps of the process and the related quantities of glycerin supplied respectively to steps (a) and (d), according to the usual stoichiometric rules.

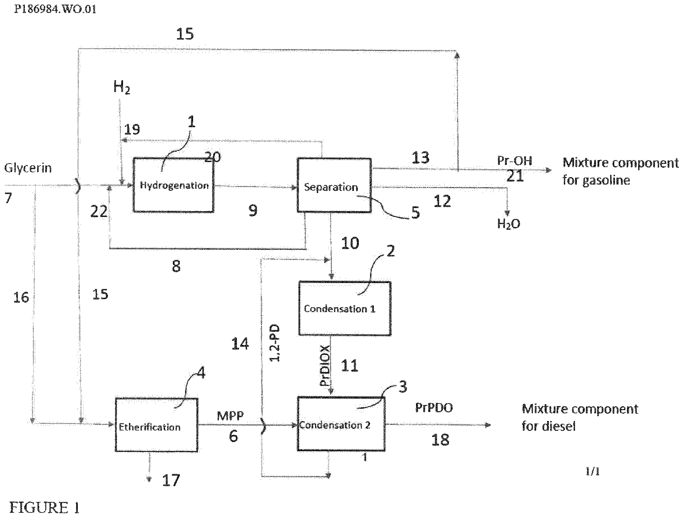

The present invention is now illustrated in particular with reference to the process diagram reported in Figure 1, without this intending to limit the overall scope of the reaction as claimed herein in any way.

With reference to Figure 1, the glycerin (7) refined with an appropriate purity grade (99%), is divided up according to the desired quantities supplied to the hydrogenation (1) and etherification reactors (4), respectively.

The glycerin (22) intended for hydrogenation can be reacted in the gaseous or liquid phase. In the first case it is mixed with a large excess of hydrogen largely coming from the recycle stream (20) and partially from the make-up supply (19). The mixture is then sent to the hydrogenation reactor in the gaseous phase (1) which operates in the following range of operating conditions: temperature: 220-275 °C; pressure: 2-4 MPa; LHSV: 0.2-0.7 h⁻¹; hydrogen/glycerin ratio: 450-700. If the glycerin reacts in the liquid phase, glycerin (22) possibly in concentrated aqueous solution, and hydrogen (19, 20) are supplied to a reactor that typically operates in the following range of operating conditions: temperature: 100-200 °C; pressure: 3-6 MPa; LHSV: 0,2-0,5 h⁻¹; hydrogen/glycerin ratio: 3-50.

The conversion of glycerin is typically greater than 70%, but can even reach almost complete conversion (99%), according to the reaction conditions, while the molar ratio (n+iso)-propanol/1,2-propanediol is preferably comprised between 1.5 and 4 and can be adjusted by a person skilled in the art using parameters such as contact time, temperature, hydrogen pressure and type of catalyst. Such a range of ratios allows the advantageous and flexible production according to market demand of both the mixture bio-components for gasoline blends and for diesel blends. The products at the outlet from the reactor are cooled (not shown in the figure) and subsequently sent with the line (9) to a separation unit (5) comprising a hydrogen separator and the distillation system of the condensed products, preferably comprising various columns. The hydrogen separated from the condensed fraction is recycled to the hydrogenation reactor (1) through the line (20), while the liquid fraction is separated by distillation through a system normally comprising a first column that operates at atmospheric pressure that separates as the last product a high boiling point mixture containing 1,2-propanediol, glycerin and possibly acetol, subsequently separated by atmospheric or vacuum distillation of the propanediol. The mixture containing water, n-propanol and isopropanol, as well as any sub-products such as ethanol, is sent, according to a well-known technique applied to similar mixtures obtained in other processes, to a distillation system comprising two interconnected columns that operate at atmospheric pressure and at reduced pressure, known as "pressure swing distillation", which allows 1,2-propanol and 1,3-propanol to be separated from their azeotropic mixture with water. A stream normally less than 5% by weight with respect to the stream supplied by line 9, is therefore separated by the unit (5) through the stream 12, which prevalently contains water and smaller quantities, usually less than 20% by weight, with respect to the actual stream, of volatile compounds such as propane, methanol and ethyl alcohol. A stream of 1,2-propanediol and a stream of 1-propanol, possibly also comprising 2-propanol, exit from the separation unit (5). The 2-propanol can be used directly as a blending component for the gasoline blend.

The 1,2-propanediol is sent to the condensation reactor (2), loaded with an acid resin, where condensation to 2-ethyl-4 methyl-1,3-dioxolane (PRDIOX) takes place. The reaction can be performed in the following range of operating conditions: Temperature: 150-200 °C; Pressure: 0.5-2 MPa; LHSV: 0.3-1.0 h⁻¹

The products exiting from the reactor are sent to a separation section (included in unit 2 in figure 1) which has the aim of separating the PRDIOX from the other components. The 1,2-propanediol is recycled to the reactor while the water is sent to the treatment section. The small quantity of propionic aldehyde that is formed during the reaction can be used together with the PRDIOX for the subsequent synthesis of 2-ethyl-4-(n-propoxymethyl)-1,3-dioxolane.

The remaining part of the glycerin coming from the line (7) is supplied through the line (16), together with the pre-selected quantity of 1-propanol (15) coming from the separation unit (5), to the reactor (4) loaded with an acid resin that operates in the following range of operating conditions:
Temperature: 110-170 °C; Pressure: 1-6 MPa; LHSV: 0.2-1 h⁻¹; propanol/glycerin (molar ratio): 2:1- 15:1

The reaction products at the outlet of the reactor are sent to the separation section (not shown in figure 1 and included in the unit (4)) from which the following are obtained:
- unreacted glycerin and propanol which are recycled to the reactor;
- water which is sent to the treatment section before discharge with the line 17;
- the 3-(n-propoxy)-1,2-propanediol which is sent (6) together with the 2-ethyl-4-methyl-1,3-dioxolane (PRDIOX) (11) to the reactor (3) for the synthesis of 2-ethyl-4-(n-propoxymethyl)-1,3-dioxolane.

The mixture of 3-propoxy-1,2-propanediol and 2-ethyl-4-methyl-1,3-dioxolane is sent to the fixed-bed reactor (3), loaded with an acid resin, which operates in the following range of operating conditions:
Temperature: 30-60 °C; pressure: 0.1-2.0 MPa; LHSV: 1-4 h-1; PRDIOX/PRPDO(ratio by weight): 1:0.5 - 1:2.

The reaction product is sent to the separation section (not shown in the figure 1) from which the following are obtained:
- The desired product 2-ethyl-4-(n-propoxymethyl)-1,3-dioxolane (18)
- The unreacted components PRDIOX and MPP which are subsequently recycled to the condensation reactor (3)
- The 1,2-propanediol which is recycled to the reactor (2) through the line (14) to form more PRDIOX.

As can be deduced from the preceding description, a particularly advantageous aspect of the process according to the present invention is comprised by its versatility. Said process allows, in variable mutual quantities as preferred, and therefore in quantities that can be directed and selected according to market requirements, both mixture components for gasolines blends and mixture components for diesel to be obtained. For example, if gasoline and diesel of fossil origin for transport are currently sold in proportions of 0.34/1 by weight, it may be desirable to produce mixture components for gasoline and diesel in the same weight proportions. The process according to the present invention advantageously allows the production of 1-propanol and 2-ethyl-4(n-propoxymethyl)-1,3-dioxolane (PrPDO) in the same proportions by weight (0.92/1 in moles) starting from glycerin only, by performing the hydrogenation of the glycerin in step (a) so that in the effluent mixture from the reactor a 1-propanol/1,2-propanediol molar ratio equal to 1.9/1 is obtained, and then dividing up the 1-propanol separated by the distillation column in the proportion of 52% supplied to the etherification step (d) of the glycerin and 48% to be used as a biocomponent for gasoline. If instead 1-propanol and PrPDO are to be produced in a ratio by weight of 1/1.03 (according to a recent projection of future trend of sales percentages of gasoline and diesel), it would be possible to change the process according to the present invention to obtain such proportions by simply changing the hydrogenation conditions of the glycerin in step (a) so that in the effluent composition from the reactor there is a 1-propanol/1,2-propanediol molar ratio of 3.6/1 and subsequently dividing up the 1-propanol separated from the distillation column in the proportion of 28% supplied to the etherification step (d) of the glycerin and 72% intended for use as a bio-component for gasoline.

For the purpose of putting the present invention into practice and illustrating it more clearly, below are some non-limitative examples of the overall scope thereof.

### EXAMPLE

### Reagents and materials

Copper chromite (Sigma-Aldrich)
Hydrogen (Sapio)
Glycerin (pure 99%, Sigma-Aldrich)
Amberlyst 36 (Dow Chemical)
Amberlyst 70 (Dow Chemical)
The glycerin is supplied in two streams to the hydrogenation step (a) and to the etherification step (d), respectively, in proportions of 4.6/1, respectively.

### EXAMPLE steps (a) and (b): Hydrogenation of glycerin to 1-propanol/1,2-propanediol and separation of the products thus obtained.

10 g of catalyst comprising copper chromite are loaded into a fixed-bed reactor. After housing the reactor in the oven and connecting it with the supply lines, nitrogen is supplied at a flow rate of 10 Nlh⁻¹ for 30 minutes and in the meantime, it is pressurized at 5 MPa. After this step has finished, hydrogen is sent at the same pressure and the reactor is heated to the temperature of 110°C. Subsequently an 80 % water-glycerin solution by weight in glycerin is supplied at a flow rate such as to have a WHSV of glycerin equal to 0.4 h⁻¹ and in the meantime the flow rate of hydrogen is adjusted so as to have a H₂/glycerin flow rate at the input to the reactor of 6. Finally, the temperature of the reactor is brought to 230°C. The reaction products at the outlet of the reactor are collected in a gas/liquid separator, maintained at the temperature of 10°C, to separate the hydrogen, which is recycled to the reactor, from the liquid phase, which is collected and analyzed through gas chromatography.

The results of the analysis of the reaction mixture highlighted:
Glycerin conversion: 68%;
molar selectivity to 1-propanol: 45%;
molar selectivity to 1,2-propanediol: 42%;
Molar selectivity to acetol: 9%;
molar selectivity to 2-propanol: 2%

The reaction mixture is subsequently supplied to a separation system comprising two fractional distillation columns in series, which separate, respectively, the first water and propanol, and the second 1,2-propanediol, leaving a residue substantially comprising unreacted glycerin and acetol, which is recycled to the hydrogenation reactor.

### EXAMPLE Step (d): Preparation of 3-progoxy-1.2-propanediol (MPP)

10 cc of Amberlyst 36 commercial resin are loaded into a fixed-bed reactor having a volume of 90 ml, and brought to the temperature of 147°C. A 1-propanol/glycerin solution with a molar ratio of 5:1, is supplied to the reactor with a spatial velocity of 0.35 h⁻¹ and at the pressure of 3 MPa. The reaction mixture at the outlet of the reactor is analyzed through GC indicating a 73.46% conversion of the glycerin and an MPP selectivity of 93%, with complement to 100% essentially comprising 2,3-dipropoxy-propan-1-ol. The catalytic system did not show any significant signs of deactivation after 1000 hours of time-on-stream. The mixture of products is subsequently subjected to distillation obtaining MPP with a purity of 98.3%. The excess propanol is recycled to the autoclave, after the separation from the sub-products and addition of glycerin and fresh make-up 1-propanol prepared according to the previous step (a), so as to maintain the 1-propanol/glycerin molar ratio equal to 5:1.

### EXAMPLE Step (c): Preparation of 2-ethyl-4-methyl 1.3 dioxolane (PrDIOX)

20 cc of Amberlyst 70 acid resin are loaded into a fixed bed. After being heated to 180°C, 1,2-propanediol is supplied at a spatial velocity of 0.5 h⁻¹. The reaction mixture is kept in the liquid phase by maintaining the pressure of the reactor at 0.7 MPa. The mixture of products at the outlet of the reactor is collected and subsequently analyzed through GC. The conversion of the 1,2-propanediol to 2-ethyl-4methyl-1,3-dioxolane is under these conditions substantially complete with a selectivity in the desired product of 96%. The complement to 100 comprises propionic aldehyde, which is not separated from the main product. The catalyst has been shown to be stable under the reaction conditions and no significant signs of deactivation have been observed after about 300 hours of time on stream.

### EXAMPLE Step (e): Synthesis of 2-ethyl-4-(n-propoxymethyl)-1,3-dioxolane from 2-ethyl-4-methyl-1.3-dioxotane PrPDO.

60 cm³ (about 46 g) of Amberlyst A-36 (Dow Chemical) acid catalyst were loaded into a fixed-bed reactor with a length of 100 cm and internal diameter of 1 cm.

After bringing the temperature of the reactor to 55°C, a mixture was supplied comprising 20% by weight of 2-ethyl-4-methyl-1,3-dioxolane (purity 88% by weight) and 80% 3-propoxy-1,2-propandiol (purity 99.3% by weight).

The mixture is supplied at the spatial velocity of 4 h⁻¹.

The reaction mixture is kept in the liquid phase by applying a back-pressure to the reactor of 4 MPa.

During the reaction, samples were taken of the mixture of products at the outlet of the reactor, which are analyzed through gas chromatography.

The gas chromatography analysis confirmed a molar conversion by step of 2-ethyl-4-methyl-1,3-dioxolane equal to 72 mol.%. The molar selectivity of the desired product 2-ethyl-4-(n-propoxymethyl)-1,3-dioxolane, is about 89 mol %.

Finally, it is however to be understood that further changes and variations may be made to the process described and illustrated herein which do not depart from the scope of the appended claims.

## Claims

1. Process for the production of propanol useful as a component, preferably bio-component, of gasoline and diesel fuel, comprising the following steps:
a) catalytic hydrogenation of glycerin, preferably obtained by biological processes, with a conversion of not less than 50% to obtain a mixture of 1-propanol and 1,2-propanediol in a molar ratio between them from 50/50 to 90/10;
b) separation of 1-propanol, 1,2-propanediol and glycerin from the reaction mixture of step (a).

2. Process according to claim 1, wherein said hydrogenation step (a) is conducted in the presence of a catalyst selected from copper chromite, mixed oxides of chromium-zinc-copper, noble metals on coal, noble metals on iron oxide.

3. Process according to any one of claims 1 or 2, wherein, in said step (a) the glycerin conversion is comprised between 60 and 99%.

4. Process according to one or more of the previous claims wherein glycerin is used containing impurities of salts, water and optionally methanol, wherein said process comprises a glycerin purification pretreatment comprising the following steps:
- removal of the salts contained in glycerin by treatment with acid exchange resins;
- removal of water and possibly methanol by fractional distillation.

5. Process according to the preceding claims wherein step (a) is performed in a single reactive step, or in two sub-steps in sequence, first substantially producing 1,2-propanediol and subsequently hydrogenating part of this to 1-propanediol.

6. Process according to claim 5, wherein when step (a) is performed in two sub-steps, they may be performed in two separate reactors or in different zones of the same reactor, in which there are specific conditions and catalysts.

7. Process according to anyone of the preceding claims wherein in step (a) glycerin is present as such, or in the presence of a solvent.

8. Process according to anyone of the preceding claims wherein step (a) is performed at a temperature comprised between 100°C and 250°C, under hydrogen pressure comprised between 0.1 and 10 MPa absolute.

9. Process according to anyone of the preceding claims, wherein the main components of the mixture obtained from step (a) are water, 1-propanol with possible smaller amounts of 2-propanol, 1,2-propanediol with possible smaller amounts of 1,3-propanediol, glycerin with any acetal formed as an intermediate.

10. Process according to anyone of the preceding claims wherein at the end of step (a) mixtures of 1-propanol and 1,2-propanediol are obtained comprising at the most up to 5% by weight with respect to the weight of 1-propanol and 1,2-propanediol produced, of 2-propanol and 1,3- propanediol, optionally comprising acetol in quantities of not more than 15% by weight of the main products, as well as any unreacted glycerin.

11. Process according to claim 10, wherein the mixture obtained after step (a) may further comprises ethylene glycol, ethanol, propane in a total amount of less than 5%, preferably less than or equal to 2% by weight.

12. Process according to anyone of the preceding claims wherein at the end of step (a) 1-propanol and 1,2-propanediol are obtained in molar ratios to each other comprised between 60/40 and 80/20, more preferably from 65/35 to 75/25.

13. Process according to anyone of the preceding claims wherein the separation step (b) of the mixture of products obtained in step (a) is performed through fractional distillation.

14. Process according to anyone of the preceding claim 1-12 wherein the separation step (b) is performed through two or more distillation columns, placed in series with each other.

15. Process according to anyone of the preceding claims 1-12, 14 wherein the separation of the reaction products of step (a) is performed using a first column that operates at atmospheric pressure that separates, as the last product, a high boiling point mixture containing 1,2-propanediol, glycerin and possibly acetol, which are subsequently separated by atmospheric or vacuum distillation of the propanediol.

16. Process according to claim 15 wherein the stream containing water, n-propanol and isopropanol, as well as any sub-products such as ethanol, are sent to a distillation system comprising two interconnected columns that operate at atmospheric pressure and at reduced pressure ("pressure swing distillation") to allow 1,2-propanol and 1,3-propanol to be separated from their azeotropic mixture with water.

17. Process according to anyone of the preceding claims, wherein the separation step (b) is carried out in a separation unit (5) comprising a hydrogen separator, the separated hydrogen being recycled to the hydrogenation reactor (1).

18. Process according to anyone one of the preceding claims, wherein the obtained 1-propanol from step (b) is supplied in part to an etherification step (d) of glycerin in the presence of at least one acid catalyst to obtain 3-(n- propoxy)-1 ,2-propanediol.
